# FASCICULE DE BREVET EUROPEEN

(11) **EP 3 311 851 B1**
(45) Date de publication et mention de la délivrance du brevet: **28.08.2019**
(21) Numéro de dépôt: 17191956.6
(22) Date de dépôt: 19.09.2017
(51) Int. Cl.: A61L 9/12, B60H 3/00

(54) **PROCÉDÉ DE DIFFUSION DE FRAGRANCE DANS L'HABITACLE D'UN VÉHICULE AUTOMOBILE**
DUFTDIFFUSIONSVERFAHREN IM INNENRAUM EINES KRAFTFAHRZEUGS
METHOD FOR DIFFUSING FRAGRANCE INTO THE PASSENGER COMPARTMENT OF A MOTOR VEHICLE

(30) Priorité: 20.10.2016 FR 1660162
(43) Date de publication de la demande: 25.04.2018
(73) Titulaire: PSA Automobiles SA, 78300 Poissy (FR)
(72) Inventeur: POCHOLLE, BENOIT, 25260 ETOUVANS (FR); GHIBAUDO, LAURENCE, 90000 BELFORT (FR); ROIZOT, DIDIER, 25600 SOCHAUX (FR)

(56) Documents cités:
- EP-A1- 2 145 788
- WO-A2-2008/050345
- FR-A1- 2 946 284
- US-A1- 2006 222 672
- US-A1- 2012 107 172

## Description

### Domaine de l'invention

La présente invention est relative aux dispositifs diffuseurs de fragrances à l'intérieur de l'habitacle des véhicules automobiles, et concerne en particulier, un procédé de diffusion pour un tel diffuseur.

### Arrière-plan de l'invention

On connaît déjà des dispositifs diffuseurs de fragrances, qui comportent le plus souvent un réceptacle muni éventuellement d'un couvercle ou d'un élément de fermeture semblable que l'utilisateur manoeuvre lorsqu'il souhaite obtenir une émission d'air parfumé, ce réceptacle comportant des moyens de suspension en un point approprié de l'habitacle, le cas échéant devant un orifice de sortie par lequel passe l'air, chaud ou froid, fourni dans cet habitacle par un ventilateur ou par simple convection naturelle.

Il est également connu d'équiper les véhicules automobiles de diffuseurs de parfum d'ambiance intégrés à l'habitacle. On trouve ainsi des dispositifs prélevant le parfum liquide dans un réservoir à l'aide d'une pompe pour le diffuser sous forme de pulvérisation dans l'habitacle. Un autre type de diffuseur est constitué d'une cartouche de parfum disposé dans un logement placé dans un élément de répartition de l'air dans l'habitacle ou en dérivation sur celui-ci. Un moyen de réglage constitué, par exemple, d'un boisseau ou d'un tiroir, permet au moins d'ouvrir ou de fermer la diffusion du parfum et parfois de régler le débit de diffusion à l'aide d'une mollette ou d'un bouton.

La demande française FR 2 867 424 décrit un tel diffuseur muni d'une cartouche dotée de plusieurs réservoirs contenant chacun un élément de stockage et de diffusion d'une fragrance, de sorte à pouvoir diffuser plusieurs parfums différents selon la demande des occupants du véhicule. L'utilisateur a également la possibilité de régler l'intensité du parfum, la zone de diffusion de ce parfum dans l'habitacle, la durée du cycle de diffusion ou encore le rapport cyclique de diffusion (c'est-à-dire le rapport exprimé en pourcentage entre la durée effective de la phase de diffusion sur un cycle et la durée totale de ce cycle).

Toutefois les commandes de ces dispositifs sont manuelles et ne permettent pas d'obtenir un paramétrage optimal, ce qui conduit à l'apparition de réactions d'accoutumance de l'utilisateur à la fragrance diffusée.

Depuis peu, et afin d'améliorer la qualité de prestation offerte, de nouveaux types de dispositifs comprenant des moyens de contrôle automatique de la diffusion de la fragrance sont apparus sur le marché. La demande américaine US 2012/0107172 divulgue ainsi un tel dispositif commandé par un processus de contrôle répétant séquentiellement les étapes suivantes :
- diffusion de la fragrance selon un cycle reproduit un nombre prédéterminé de fois et comprenant une phase de diffusion suivi d'une phase de pause,
- arrêt de la diffusion du parfum sous forme cyclique durant une période de temporisation prédéterminée.

La durée effective de la phase de diffusion sur chaque cycle est déterminée en fonction de la température intérieure de l'air régnant dans l'habitacle et de certains paramètres du système de conditionnement d'air du véhicule.

Bien que les risques d'accoutumance des usagers à la fragrance soient réduits, la perception olfactive de la fragrance diffusée et ses effets relaxants induits diminuent sensiblement avec le temps. On connait aussi des systèmes pour contrôler la diffusion des fragrances, tels que décrits dans les demandes EP 2145788 A1, US 2006/0222672 A1, FR 2946284 et WO 2008/050345 A2.

### Objet et résumé de l'invention

La présente invention vise donc à optimiser la perception olfactive de la fragrance diffusée par l'utilisateur

Elle propose à cet effet un procédé de diffusion de fragrance dans l'habitacle d'un véhicule automobile pour diffuseur du type comportant une cartouche amovible dotée d'au moins un réservoir contenant une dite fragrance, l'un ou plusieurs des paramètres de diffusion de ladite fragrance variant en fonction de la température intérieure régnant dans l'habitacle et de certains paramètres du système de conditionnement d'air dudit véhicule, dans lequel au moins l'un desdits paramètres de diffusion de ladite fragrance varie en fonction de la durée de diffusion cumulée de ladite fragrance depuis la mise en place de ladite cartouche ; la diffusion de ladite fragrance étant réalisée suivant un cycle de diffusion par intermittence se répétant en boucle, ledit cycle étant constitué de deux phases successives de durées prédéterminées : une première phase de diffusion pendant laquelle ladite fragrance est diffusée dans l'habitacle, et une seconde phase de pause durant laquelle ladite fragrance n'est pas diffusée dans l'habitacle, ledit procédé étant caractérisé en ce que la durée de ladite phase de diffusion décroit avec l'augmentation de ladite température intérieure régnant dans l'habitacle.

En prenant en compte la perte progressive de concentration de la fragrance contenue dans le réservoir de la cartouche amovible, le procédé de diffusion selon l'invention permet d'obtenir en permanence un ressenti olfactif équilibré pour les passagers et d'optimiser ainsi les effets relaxants induits par la diffusion de la fragrance.

Selon des caractéristiques préférées du procédé, prises seules ou en combinaison :
- la diffusion de ladite fragrance est réalisée suivant un cycle de diffusion par intermittence se répétant en boucle, ledit cycle étant constitué de deux phases successives de durées prédéterminées : une première phase de diffusion pendant laquelle ladite fragrance est diffusée dans l'habitacle, et une seconde phase de pause durant laquelle ladite fragrance n'est pas diffusée dans l'habitacle ;
- la durée de ladite phase de diffusion croit avec l'augmentation de ladite durée de diffusion cumulée ;
- la durée de ladite phase de pause croit avec l'augmentation de ladite température intérieure régnant dans l'habitacle ;
- la durée de ladite phase de pause croit avec l'augmentation du débit d'air délivré dans l'habitacle par ledit système de conditionnement d'air ;
- la durée de ladite phase de pause varie en fonction de la répartition de l'air délivré dans l'habitacle par ledit système de conditionnement d'air ;
- la durée de ladite phase de pause croit avec l'augmentation du taux de recyclage de l'air habitacle ;
- la durée de ladite phase de pause croit avec l'augmentation de la température de l'air conditionné délivré par ledit système de conditionnement d'air ; et/ou
- ladite température intérieure régnant dans l'habitacle est estimée à partir de mesures de la température extérieure et du niveau d'ensoleillement réalisées par des capteurs.

### Brève description des dessins

L'exposé de l'invention sera maintenant poursuivi par la description détaillée d'un exemple de réalisation, donnée ci-après à titre illustratif mais non limitatif, en référence aux dessins annexés, sur lesquels :
- la figure 1 représente un schéma fonctionnel d'un dispositif de diffusion de fragrance dans l'habitacle d'un véhicule automobile, apte à mettre en oeuvre le procédé selon l'invention ; et
- la figure 2 illustre la manière dont est diffusée la fragrance dans l'habitacle du véhicule ; et
- la figure 3 représente un organigramme du procédé selon l'invention.

### Description détaillée d'un mode de réalisation

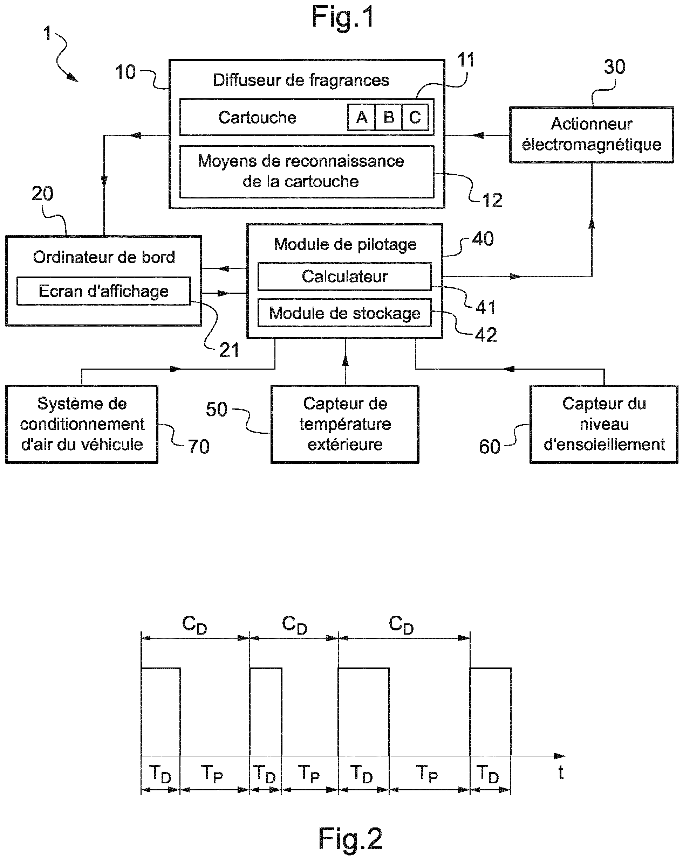

En référence à la figure 1, le dispositif de diffusion 1 comporte un diffuseur de fragrance 10 apte à diffuser de manière autonome du parfum dans l'habitacle du véhicule au travers d'au moins un passage en forme de fente ménagé dans la paroi frontale de la planche de bord de ce véhicule.

Ce diffuseur de fragrance 10 comprend un logement d'accueil pour une cartouche amovible 11 dotée de plusieurs réservoirs A, B, C contenant chacun une fragrance.

Le diffuseur 10 comporte également des moyens de reconnaissance 12 de la cartouche 11 permettant d'identifier sa référence exacte, chaque référence correspondant à un arrangement spécifique de différentes fragrances contenues dans les réservoirs A, B et C.

Le diffuseur 10 est relié à l'ordinateur de bord 20 du véhicule auquel il transmet la référence de la cartouche 11 insérée dans son logement. En fonction de cette référence et d'un tableau de correspondance stocké en mémoire, l'ordinateur de bord 20 peut ainsi déterminer la fragrance contenue dans chacun des réservoirs A, B, C de cette cartouche 11.

Un mode paramétrage permet à l'utilisateur de sélectionner, à l'aide d'un sélecteur ou d'un bouton de commande, la fragrance à diffuser parmi les fragrances qui lui sont proposées sur l'écran d'affichage 21 de l'ordinateur de bord 20 et qui correspondent à celles contenues dans les réservoirs A, B, C de la cartouche 11.

Chaque réservoir A, B, C comporte un orifice dont l'ouverture et la fermeture sont gérés par un mécanisme d'obturation tel qu'une vanne, un boisseau ou encore un tiroir, de sorte à autoriser ou non la diffusion de la fragrance qu'il contient dans l'habitacle du véhicule.

Ce mécanisme d'obturation est commandé par un actionneur électromagnétique 30, lui-même piloté par un module de pilotage 40 qui gère la diffusion de la fragrance sélectionnée par l'usager suivant un cycle de diffusion C_{D} par intermittence se répétant en boucle tant que le dispositif de diffusion est maintenu en fonctionnement, comme illustré par la figure 2.

Chaque cycle C_{D} se compose de deux phases successives : une première phase de diffusion T_{D} pendant laquelle la fragrance est diffusée dans l'habitacle (l'orifice du réservoir contenant cette fragrance est alors ouvert), et une seconde phase de pause T_{P} durant laquelle la fragrance n'est pas diffusée (l'orifice du réservoir contenant cette fragrance étant alors fermé).

Un compteur de temps intégré au module de pilotage 40 permet de mesurer la durée de diffusion cumulée de chaque fragrance contenue dans un réservoir de la cartouche 11 depuis l'installation de cette cartouche (cette durée de diffusion cumulée s'incrémentant durant chaque phase d'ouverture du réservoir contenant la fragrance).

Le module de pilotage 40 détermine pour chaque cycle C_{D} les durées des phases de diffusion T_{D} et de pause T_{P} en fonction des données transmises en continu par les capteurs 50, 60 (température extérieure et niveau d'ensoleillement) et celles en provenance du système de conditionnement d'air 70 du véhicule (débit, répartition et température de l'air délivré dans l'habitacle, taux de recyclage de l'air habitacle).

Pour ce faire, ce module de pilotage 40 comporte un calculateur 41 et un module de stockage 42 qui comprend de la mémoire non volatile de type EEPROM ou FLASH et de la mémoire vive.

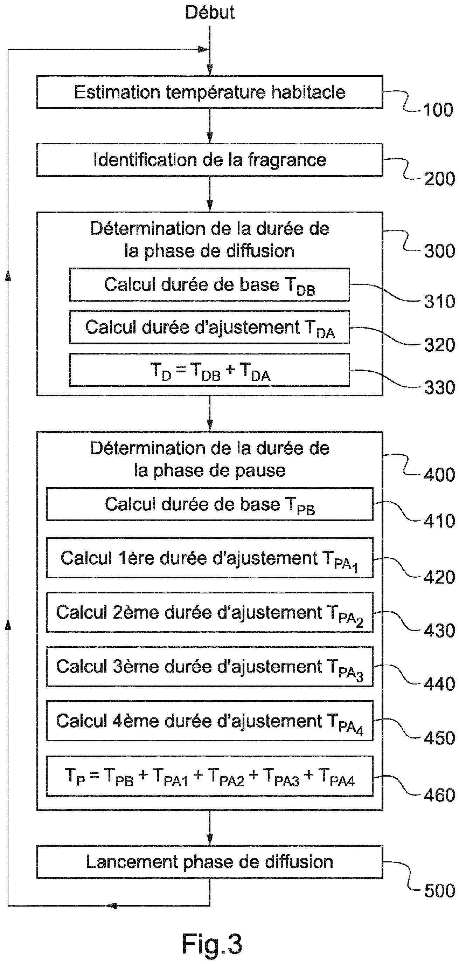

La mémoire non volatile stocke un processus de diffusion de fragrance qui est mis en oeuvre dans ce module 40 et dont l'organigramme est représenté sur la figure 3.

Le module de pilotage 40 est également relié à l'ordinateur de bord 20. En particulier ce dernier indique au module 40 le type de fragrance sélectionnée par l'utilisateur, tandis que le module 30 lui transmet les informations relatives à la diffusion de la fragrance afin qu'elles apparaissent sur l'écran d'affichage 21.

On va maintenant décrire en détails et à l'appui de l'organigramme de la figure 3, les différentes étapes de ce processus qui se lance automatiquement lorsqu'un passager du véhicule demande via une commande de l'ordinateur de bord la diffusion de fragrance dans l'habitacle.

Le module de pilotage commence par estimer la température régnant à l'intérieur de l'habitacle à partir de la température extérieure et du niveau d'ensoleillement mesurés par les capteurs (étape 100).

Cette estimation est par exemple obtenue à partir d'une formule de calcul ou d'un tableau de correspondance à double entrée stocké en mémoire dans le module 42 et représentant la température interne en fonction de la température extérieure et du niveau d'ensoleillement.

Le module de pilotage identifie ensuite la fragrance sélectionnée à partir des informations transmises par l'ordinateur de bord (étape 200).

Le calculateur va alors déterminer, respectivement au cours des étapes 300 et 400, les durées des phases de diffusion et de pause du prochain cycle.

Le calcul de la durée de la phase de diffusion T_{D} intervenant au cours de l'étape 300 s'effectue en plusieurs sous étapes successives.

Au cours d'une première sous-étape 310, le module de pilotage 40 détermine une durée de base T_{DB} pour cette phase de diffusion T_{D} en fonction de la température intérieure précédemment estimée.

Afin de compenser la plus forte volatilité des molécules de fragrance dans de l'air chaud, cette durée de base T_{DB} décroit avec l'augmentation de la température intérieure.

Elle est obtenue à partir d'une formule de calcul ou d'un tableau de correspondance donnant la durée de la phase de diffusion en fonction de la température intérieure.

Afin de tenir compte de la différence de niveau d'intensité olfactive entre les différentes fragrances contenues dans les reservoirs de la cartouche, le module de pilotage 40 utilisera avantageusement une formule de calcul ou un tableau de correspondance spécifique pour chaque fragrance.

Le processus va ensuite déterminer, lors de la sous-étape 320, la durée d'ajustement à ajouter à cette durée de base T_{DB} de la phase de diffusion pour compenser la perte progressive de concentration de la fragrance.

Cette durée d'ajustement T_{DA}, obtenue via une formule de calcul ou un tableau de correspondance stocké en mémoire, croit (avantageusement de manière linéaire ou par palier) avec l'augmentation de la durée de diffusion cumulée de la fragrance sélectionnée depuis la mise en place de la cartouche 11 dans le diffuseur 10.

La durée de la phase de diffusion T_{D} est alors obtenue en additionnant la durée de base T_{DB} calculée à la sous-étape 310 à la durée d'ajustement T_{DA} calculée à la sous étape 320 (sous-étape 330).

La détermination de la durée de la phase de pause T_{P} intervenant au cours de l'étape 400 s'effectue également en plusieurs sous étapes successives.

Au cours d'une première sous-étape 410, le module de pilotage 40 détermine une durée de base T_{PB} pour cette phase de pause en fonction de la température intérieure précédemment estimée.

Cette durée de base T_{PB}, qui croit avec l'augmentation de la température intérieure, est obtenue à partir d'une formule de calcul ou d'un tableau de correspondance.

Afin de tenir compte de la différence de niveau d'intensité olfactive entre les différentes fragrances contenues dans les réservoirs de la cartouche 11, le module de pilotage 40 utilisera avantageusement une formule de calcul ou un tableau de correspondance spécifique pour chaque fragrance.

Le processus va ensuite déterminer, lors des étapes 420, 430, 440 et 450, des durées d'ajustement T_{PA1}, T_{PA2}, T_{PA3}, T_{PA4} à ajouter ou à retrancher à la durée de base T_{PB} de la phase de pause pour compenser les perturbations générées par le système de conditionnement d'air 70 du véhicule.

A l'étape 420, une première durée d'ajustement T_{PA1} positive (c'est-à-dire s'ajoutant à la durée de base T_{PB}) est calculée afin de prendre en compte le débit d'air délivré par le système de conditionnement d'air 70.

Cette première durée d'ajustement T_{PA1}, obtenue via une formule de calcul ou un tableau de correspondance stocké en mémoire, croit (avantageusement de manière linéaire ou par palier) avec l'augmentation de ce débit (le ressenti olfactif diminuant d'autant plus lentement que le débit d'air délivré est important).

Lors de l'étape 430, le module de pilotage 40 détermine une deuxième durée d'ajustement T_{PA2} positive ou négative (c'est-à-dire s'ajoutant ou se retranchant à la durée de base T_{PB}) afin de prendre en compte la répartition dans l'habitacle de l'air délivré par le système de conditionnement d'air 70.

Cette deuxième durée d'ajustement T_{PA2}, obtenue via une formule de calcul ou un tableau de correspondance stocké en mémoire, sera par exemple négative dans les configurations où l'air conditionné est délivré essentiellement par les buses de sortie inférieures implantées au niveau des pieds des passagers avant du véhicule (le ressenti olfactif diminuant alors rapidement après l'arrêt de la phase de diffusion de la fragrance).

Inversement, cette même durée sera positive dans les configurations où l'air conditionné est délivré essentiellement par les buses de sortie supérieures implantées au niveau de la planche de bord du véhicule (le ressenti olfactif diminuant alors plus lentement après l'arrêt de la phase de diffusion de la fragrance).

A l'étape 440, une troisième durée d'ajustement T_{PA3} positive (c'est-à-dire s'ajoutant à la durée de base T_{PB}) est calculée afin de prendre en compte le taux de recyclage de l'air habitacle.

Cette troisième durée d'ajustement T_{PA3}, obtenue via une formule de calcul ou un tableau de correspondance stocké en mémoire, croit (avantageusement de manière linéaire ou par palier) avec l'augmentation de ce taux (le ressenti olfactif diminuant d'autant plus lentement que l'air habitacle n'est pas ou peu renouvelé).

Lors de l'étape 450, le module de pilotage 40 détermine une quatrième durée d'ajustement T_{PA4} positive (c'est-à-dire s'ajoutant à la durée de base T_{PB}) afin de prendre en compte la température de l'air conditionné délivré dans l'habitacle par le système.

Cette quatrième durée d'ajustement T_{PA1}, obtenue via une formule de calcul ou un tableau de correspondance stocké en mémoire, croit (avantageusement de manière linéaire ou par palier) avec l'augmentation de cette température (le ressenti olfactif diminuant d'autant plus lentement que la température de l'air conditionné délivré dans l'habitacle est importante).

La durée de la phase de pause est alors obtenue en additionnant la durée de base calculée à la sous-étape 410 aux différentes durées d'ajustement calculées aux sous-étapes 420, 430, 440 et 450 (sous-étape 460).

Une fois les durées des phases de diffusion T_{DB} et de pause T_{PB} connues, le module de pilotage 40 lance le cycle de diffusion de la fragrance sélectionnée qui démarre par la phase de diffusion et se poursuit par celle de pause (étape 500).

Au terme de ce cycle, le processus se répète automatiquement à partir de l'étape initiale 100.

Selon des variantes de réalisation non représentées, la température intérieure est obtenue directement à partir d'un capteur ménagé dans l'habitacle du véhicule.

## Revendications

1. Procédé de diffusion de fragrance dans l'habitacle d'un véhicule automobile pour diffuseur du type comportant une cartouche amovible (11) dotée d'au moins un réservoir contenant une dite fragrance, l'un ou plusieurs des paramètres de diffusion (T_{D}, T_{P}) de ladite fragrance variant en fonction de la température intérieure régnant dans l'habitacle et de certains paramètres du système de conditionnement d'air dudit véhicule (70), dans lequel au moins l'un (T_{D}, T_{P}) desdits paramètres de diffusion de ladite fragrance varie en fonction de la durée de diffusion cumulée de ladite fragrance depuis la mise en place de ladite cartouche (11) ; la diffusion de ladite fragrance étant réalisée suivant un cycle de diffusion (C_{D}) par intermittence se répétant en boucle, ledit cycle étant constitué de deux phases successives de durées prédéterminées : une première phase de diffusion (T_{D}) pendant laquelle ladite fragrance est diffusée dans l'habitacle, et une seconde phase de pause (T_{P}) durant laquelle ladite fragrance n'est pas diffusée dans l'habitacle, ledit procédé étant **caractérisé en ce que** la durée de ladite phase de diffusion (T_{D}) décroit avec l'augmentation de ladite température intérieure régnant dans l'habitacle.

2. Procédé de diffusion de fragrance selon la revendication précédente, **caractérisé en ce que** la durée de ladite phase de diffusion (T_{D}) croit avec l'augmentation de ladite durée de diffusion cumulée.

3. Procédé de diffusion de fragrance selon l'une des revendications précédentes, **caractérisé en ce que** la durée de ladite phase de pause (T_{P}) croit avec l'augmentation de ladite température intérieure régnant dans l'habitacle.

4. Procédé de diffusion de fragrance selon l'une des revendications précédentes, **caractérisé en ce que** la durée de ladite phase de pause (T_{P}) croit avec l'augmentation du débit d'air délivré dans l'habitacle par ledit système de conditionnement d'air.

5. Procédé de diffusion de fragrance selon l'une des revendications précédentes, **caractérisé en ce que** la durée de ladite phase de pause (T_{P}) varie en fonction de la répartition de l'air délivré dans l'habitacle par ledit système de conditionnement d'air.

6. Procédé de diffusion de fragrance selon l'une des revendications précédentes, **caractérisé en ce que** la durée de ladite phase de pause (T_{P}) croit avec l'augmentation du taux de recyclage de l'air habitacle.

7. Procédé de diffusion de fragrance selon l'une des revendications précédentes, **caractérisé en ce que** la durée de ladite phase de pause (T_{P}) croit avec l'augmentation de la température de l'air conditionné délivré par ledit système de conditionnement d'air.

8. Procédé de diffusion de fragrance selon l'une des revendications précédentes, **caractérisé en ce que** ladite température intérieure régnant dans l'habitacle est estimée à partir de mesures de la température extérieure et du niveau d'ensoleillement réalisées par des capteurs.

## Patentansprüche

1. Verfahren zur Diffusion von Duft in dem Innenraum eines Kraftfahrzeugs für Verteiler vom Typ, der eine abnehmbare Patrone (11) umfasst, die mit mindestens einem Behälter versehen ist, der einen Duft enthält, wobei ein oder mehrere der Diffusionsparameter (T_{D}, T_{P}) des Dufts in Abhängigkeit von der Innentemperatur, die in dem Innenraum herrscht, und von bestimmten Parametern der Klimaanlage des Fahrzeugs (70) variieren, wobei mindestens einer der Diffusionsparameter (T_{D}, T_{P}) des Dufts in Abhängigkeit von der kumulierten Diffusionsdauer des Dufts ab dem Anbringen der Patrone (11) variiert; wobei das Verteilen des Dufts gemäß einem Diffusionszyklus (C_{D}), der sich in einer Schleife wiederholt, intermittierend ausgeführt wird, wobei der Zyklus aus zwei aufeinanderfolgenden Phasen mit vorbestimmten Dauern besteht: eine erste Diffusionsphase (T_{D}), während der der Duft in dem Innenraum verteilt wird, und eine zweite Pausephase (T_{P}), während der der Duft in dem Innenraum nicht verteilt wird, Verfahren **dadurch gekennzeichnet, dass** die Dauer der Diffusionsphase (T_{D}) mit der Erhöhung der Innentemperatur, die in dem Innenraum herrscht, sinkt.

2. Duftdiffusionsverfahren nach dem vorstehenden Anspruch, **dadurch gekennzeichnet, dass** die Dauer der Diffusionsphase (T_{D}) mit der Erhöhung der kumulierten Diffusionsdauer steigt.

3. Duftdiffusionsverfahren nach einem der vorstehenden Ansprüche, **dadurch gekennzeichnet, dass** die Dauer der Pausephase (T_{P}) mit der Erhöhung der Innentemperatur, die in dem Innenraum herrscht, steigt.

4. Duftdiffusionsverfahren nach einem der vorstehenden Ansprüche, **dadurch gekennzeichnet, dass** die Dauer der Pausephase (T_{P}) mit der Erhöhung des Luftdurchsatzes, der in den Innenraum von der Klimaanlage geliefert wird, steigt.

5. Duftdiffusionsverfahren nach einem der vorstehenden Ansprüche, **dadurch gekennzeichnet, dass** die Dauer der Pausephase (T_{P}) in Abhängigkeit von der Diffusion der Luft, die von der Klimaanlage in den Innenraum geliefert wird, variiert.

6. Duftdiffusionsverfahren nach einem der vorstehenden Ansprüche, **dadurch gekennzeichnet, dass** die Dauer der Pausephase (T_{P}) mit der Erhöhung der Recyclingrate der Innenraumluft variiert

7. Duftdiffusionsverfahren nach einem der vorstehenden Ansprüche, **dadurch gekennzeichnet, dass** die Dauer der Pausephase (T_{P}) mit der Erhöhung der Temperatur der aufbereiteten Luft, die von der Klimaanlage geliefert wird, steigt.

8. Duftdiffusionsverfahren nach einem der vorstehenden Ansprüche, **dadurch gekennzeichnet, dass** die Innentemperatur, die in dem Innenraum herrscht, ausgehend von Messungen der Außentemperatur und des Sonnenbestrahlungsniveaus, die von Fühlern ausgeführt werden, geschätzt wird.

## Claims

1. A method for diffusing fragrance into the passenger compartment of a motor vehicle for a diffuser of the type comprising a removable cartridge (11) equipped with at least one reservoir containing a said fragrance, one or several of the diffusion parameters (T_{D}, T_{P}) of said fragrance varying as a function of the interior temperature prevailing in the passenger compartment and of certain parameters of the air conditioning system of said vehicle (70), in which at least one (T_{D}, T_{P}) of said diffusion parameters of said fragrance varies as a function of the cumulative diffusion duration of said fragrance from the putting in place of said cartridge (11); the diffusion of said fragrance being realized according to a diffusion cycle (C_{D}) by intermittence, repeating in a loop, said cycle being constituted by two successive phases of predetermined durations: a first diffusion phase (T_{D}) during which said fragrance is diffused in the passenger compartment, and a second pause phase (T_{P}) during which said fragrance is not diffused in the passenger compartment, said method being **characterized in that** the duration of said diffusion phase (T_{D}) decreases with the increase of said interior temperature prevailing in the passenger compartment.

2. The method for diffusing fragrance according to the preceding claim, **characterized in that** the duration of said diffusion phase (T_{D}) increases with the increase of said cumulative duration of diffusion.

3. The method for diffusing fragrance according to one of the preceding claims, **characterized in that** the duration of said pause phase (T_{P}) increases with the increase of said interior temperature prevailing in the passenger compartment.

4. The method for diffusing fragrance according to one of the preceding claims, **characterized in that** the duration of said pause phase (T_{P}) increases with the increase of the flow of air delivered in the passenger compartment by said air conditioning system.

5. The method for diffusing fragrance according to one of the preceding claims, **characterized in that** the duration of said pause phase (T_{P}) varies as a function of the distribution of the air delivered in the passenger compartment by said air conditioning system.

6. The method for diffusing fragrance according to one of the preceding claims, **characterized in that** the duration of said pause phase (T_{P}) increases with the increase of the recycling rate of the passenger compartment air.

7. The method for diffusing fragrance according to one of the preceding claims, **characterized in that** the duration of said pause phase (T_{P}) increases with the increase of the temperature of the conditioned air delivered by said air conditioning system.

8. The method for diffusing fragrance according to one of the preceding claims, **characterized in that** said interior temperature prevailing in the passenger compartment is estimated from measurements of the exterior temperature and of the level of sunlight carried out by sensors.
